# EUROPEAN PATENT APPLICATION

(11) **EP 3 984 376 A1**
(43) Date of publication of application: **20.04.2022**
(21) Application number: 21202738.7
(22) Date of filing: 14.10.2021
(51) Int. Cl.: A23K 50/10, A23K 50/60, A23K 10/28, A23K 10/14, A23K 20/163, A23C 9/18, A23C 9/12

(54) **LACTASE FOR USE**

(30) Priority: 16.10.2020 NL 2026691; 28.05.2021 NL 2028319
(71) Applicant: Nukamel NV, 2440 Geel (BE)
(72) Inventor: GOOSSENS, Evy, 9185 Wachtebeke (BE); DUCATELLE, Richard Victor Aimé, 9790 Wortegem-Petegem (BE); DERIX, Jill, 9820 Merelbeke (BE); CROES, Evi, 3545 Halen (BE)
(74) Representative: Nederlandsch Octrooibureau

(57) **Abstract**

The present invention related to lactase for preventing and/or stimulating active immunity against *Clostridium perfringens* related alimentary tract conditions, especially necro-haemorrhagic enteritis. That is to say, the invention relates to lactase for use in preventing an alimentary tract disorder caused by *Clostridium perfringens* in a bovine, preventing a *Clostridium perfringens* alpha-toxin related alimentary tract disorder in a bovine, increasing active immunity against an alimentary tract disorder in a bovine caused by *Clostridium perfringens,* increasing active immunity against a *Clostridium perfringens* alpha-toxin related alimentary tract disorder in a bovine and stimulating *Clostridium perfringens* alpha-toxin antibody production in a bovine, wherein the lactase is orally administered to the bovine subject. The invention also relates to use of lactase for preventing a bovine alimentary tract disorder caused by *Clostridium perfringens,* preventing a *Clostridium perfringens* alpha-toxin related alimentary tract disorder in a bovine, increasing active immunity against an alimentary tract disorder in a bovine caused by *Clostridium perfringens,* increasing active immunity against a *Clostridium perfringens* alpha-toxin related alimentary tract disorder in a bovine, and stimulating *Clostridium perfringens* alpha-toxin antibody production in a bovine, wherein the lactase is orally administered to the bovine subject. In addition, the invention relates to a method for providing a dairy-based milk replacer formulation for bovines comprising lactase.

## Description

### Field of the invention

The present invention relates to lactase and its use in preventing *Clostridium perfringens* related alimentary tract conditions in bovines.

### Background art

Milk replacers have been developed as a tool to improve daily (weight) gain in calves and to save valuable milk products for human consumption, while at the same time using less costly ingredients that are less suitable for human consumption. For these milk replacers a relative high protein (24-28 wt.%) level is deemed beneficial for growth. However intensive feeding schemes making use of these milk replacers increase the risk for gut health issues, such as ruminitis and ruminal acidosis, bloat (ruminal and abomasal), dysbiosis (with enteritis and diarrhea) and, most importantly, necro-haemorrhagic enteritis.

Necro-haemorrhagic enteritis, also known as 'the over-eating disease' and often named enterotoxaemia, is a very costly disease as it is highly fatal and affects the best growing animals at an age (approximately 15 weeks) at which they already have a high economic value. Mortality rates of 2-3% have been recorded, whilst for some breeds the mortality rate can go up to 10%. This syndrome is characterized by relatively low morbidity but extremely high mortality. Seemingly healthy calves die suddenly from this disease and experience massive intestinal bleeding. Therefore medical interventions after an animal shows signs of the disease are most often too late. Unfortunately, so far also no adequate preventive therapy is available.

Necro-haemorrhagic enteritis is deemed to be related to a high protein diet and to (intestinal growth of) *Clostridium perfringens* (Valgaeren et al., Journal of Comparative Pathology 149, 103-112, 2012). It was clearly shown that the presence of undigested milk replacer in the small intestinal lumen together with presence of *Clostridium perfringens* is a prerequisite for the development of necro-haemorrhagic enteritis. Only for this combination symptoms developed in a calf intestinal loop model. Injection of loops with milk replacer only, or with *Clostridium perfringens* only, rarely resulted in the development of a necro-hemorrhagic lesions. The strains of *Clostridium perfringens* that were isolated from field cases of necro-haemorrhagic enteritis were in no way superior in vitro toxin producers, compared to strains of *Clostridium perfringens* isolated from healthy calves or isolated from other sources (Goossens et al., BMC Veterinary Research 2014, 10:32). Moreover, using the calf intestinal loop model, it was shown that any strain of *Clostridium perfringens* was capable of inducing the necro-hemorrhagic intestinal lesions, provided it was injected together with the milk replacer. Using alpha toxin and perfringolysin knock-out mutants and complemented mutants it was shown that the necro-hemorrhagic lesions are caused by the combined action of two toxins produced by *Clostridium perfringens,* i.e. alpha toxin and perfringolysin, attacking the endothelial cells of the host (Verherstraeten et al., Veterinary Research 2013, 44:45). Both these toxins are produced by all strains of *Clostridium perfringens,* albeit in varying concentrations, i.e. to a different extent. Not only can different strains produce different concentrations of the toxins, but also one and the same strain can produce different amounts of toxins depending on environmental conditions. *Clostridium perfringens* strains that only produce alpha toxin and perfringolysin, and none of the other major toxins, are classified as type A strains.

*C*. *perfringens* alpha toxin and immunity development against this toxin was found to be key for development of the disease, i.e. necro-haemorrhagic enteritis. It was shown that traditionally weaned calves present a smooth transition from passive (maternal) humoral immunity to acquired active immunity against the alpha toxin of *Clostridium perfringens* (Valgaeren et al., Toxins 2015, 7, 2586-2597). Indeed, as *Clostridium perfringens* is a ubiquitous microorganism which is present in the intestinal tract of (almost) all animals, spontaneous development of an active acquired protective immunity is expected to occur in healthy animals under normal conditions. This is, however, in contrast to calves maintained for a relatively long time on high amounts of milk replacer, which apparently show poor seroconversion against alpha toxin when maternal antibodies are fading away, leaving a so-called immunity gap (Valgaeren et al., Toxins 2015, 7, 2586-2597). This immunity gap may be an important contributing factor in the increased susceptibility of veal calves to necro-haemorrhagic enteritis.

Vaccination so far has demonstrated not suitable to prevent necro-haemorrhagic enteritis. It is well known that the protective antigenicity of alpha toxin is easily destroyed by formaldehyde inactivation, explaining why the current clostridial vaccines are unable to protect against necro-haemorrhagic enteritis. Inactivation is needed as alpha toxin is a potent dermonecrotic toxin that is not safe for use in calves.

Milk replacer appeared to be an important predisposing component as an additive to the *C*. *perfringens* inoculum to induce typical necrohaemorrhagic lesions in the intestinal loops. In loop models developed for different clostridial diseases in different animal species, the addition of a nutrient-rich supplement was a prerequisite to induce lesions. Milk replacer contains large amounts of high-quality protein, which may stimulate the growth of *C*. *perfringens,* a bacterium that is auxotrophic for multiple amino acids. The need to add a milk replacer fits with information from the veal industry, where more necro-haemorrhagic enteritis cases occur after intake of large quantities of milk replacer without a proper adaptation period. Moreover, milk replacers containing a high amount of animal protein are typically used in the Belgian blue veal industry, which has a high prevalence of necro-haemorrhagic enteritis (9.75%). In contrary, in the rose veal industry, where a replacer with a higher amount of vegetable protein is used, necro-haemorrhagic enteritis has a low prevalence (0.89%) (Thesis B. Valgareren 2015, Ghent University).

In conclusion, necro-haemorrhagic enteritis is believed to relate to an underdeveloped active immunity in calves against *C*. *perfringens* and *C*. *perfringens* alpha-toxin specifically. This underdeveloped active immunity may lead to sudden and rapid *C*. *perfringens* intestinal overgrowth and subsequent death.

### Summary of the invention

There is a need for preventive measures and means for saving bovines from developing necro-haemorrhagic enteritis, and subsequently dying as a consequence thereof. There is a need for stimulating active immunity build-up against *C*. *perfringens* in young bovines, as a poor active immunity may lead to *C*. *perfringens* related bovine alimentary tract disorders. There is a need of preventing occurrence of these disorders and especially necro-haemorrhagic enteritis. The inventors now surprisingly found that by administering lactase to a bovine, active immunity build up against *C*. *perfringens* related alimentary tract conditions is improved. In the state of the art, *C*. *perfringens* related bovine alimentary tract disorders are deemed related to the protein in the milk replacers. In an *in-vitro* experiment the inventors demonstrated that supplements comprising lactose in an *in-vitro* assay did not affect the growth rate of *C*. *perfringens* but did lower the expression of *C*. *perfringens* alpha-toxin. Therefore, the inventors believe that lactose suppresses toxin expression by *C*. *perfringens* that is present in low amounts in the alimentary tract of young bovines, and therewith prevents the build-up of active immunity in these bovines. Due to the lagging active immunity build-up the bovine is more susceptible for *C*. *perfringens* related diseases. The *in vitro* results were confirmed in *in vivo* experiments in which it was demonstrated that by providing lactase to the bovine and therewith lowering the lactose the bovine received, active immunity build-up was improved. Moreover, the concentration of *C*. *perfringens* in the alimentary tract of the calves was not affected by the dietary intervention.

When maternally fed, calves receive milk with a dry matter content of about 12 wt.% and a lactose content of about 35 wt.% based on the total dry matter of the milk. The lactose is split by lactase that is present in the alimentary tract and the resulting mono-carbohydrates are mainly taken up in the duodenum and jejunum and only a minor part reaches the first part of the ileum and is taken up there. Bovine stomachs have a large capacity to take up mono-carbohydrates such as glucose and galactose. Upon aging, it is assumed that the lactase content in the alimentary tract of the calf decreases, therewith decreasing the enzyme-driven lactose-split capacity. The inventors believe that for the intensive feeding schemes making use of milk replacers, the amount of lactose presented to the calf is higher than the amount of lactose that the intestinal lactose processing capacity of the calf is able to sufficiently process, resulting in a significant amount of lactose in the small intestine of the calf. This intestinal lactose prevents toxin formation by *C*. *perfringens* and therefore prevents the build-up of active immunity. Typically, milk replacers are fed with a dry matter content of 13-16 wt.% and a lactose content of about 45 wt.%.

In the art the administration of lactase to certain mammals has been known to improve lactose break down. For example health claims on lactase as a dietary supplement in humans have been approved by the European Food Safety Authority (Foods, Nutrients and Food Ingredients with Authorised EU Health Claims, Woodhead Publishing, 2014, p 177-211 and EFSA Journal 2009; 7(9):1236). These claims relate to that for lactose-intolerant individuals, so subjects who are unable to break-down lactose and experience adverse effects such as bloating because of that, it was demonstrated that supplementing lactase to the individual results in digestion of lactose in the lactose-intolerant individual. Studies underlying these health claims demonstrated a positive effect of lactase supplementation on H₂ excretion and reducing or preventing the adverse effects of lactose-intolerance (Montalto et al, World Journal Gastroenterology (2006) 12(2):187-191 and Montalto et al, European Journal of Clinical Nutrition (2005) 59, 489-493). Measuring H₂ excretion is a standard test for diagnosis of lactose maldigestion. Also WO 0013526 demonstrates that providing lactase to an animal is effective in breaking down lactose that is fed. The lactase can both be provided as a supplement or mixed in a lactose comprising product such as milk.

Young bovines are fed lactose rich diets through maternal feeding and/or through feeding artificial milk replacers. The inventors now found that reducing the amount of lactose reaching the small intestine in bovines improves active immunity build-up towards *C*. *perfringens* related alimentary tract disorders, especially *C*. *perfringens* alpha-toxin related alimentary tract disorders, and thereby prevents alimentary tract disorders caused by *C*. *perfringens.* The inventors further demonstrated that reduction of this amount of lactose can be obtained by administering lactase which breaks down the lactose. Also the art confirms the effectiveness of lactase in lactose digestion both as supplement or provided to a lactose comprising diet. The invention therefore concerns administering lactase to a bovine subject. Therewith, active immunity build up towards *C*. *perfringens* is stimulated, more in particular towards alpha toxin, and alimentary tract conditions related to *C*. *perfringens* are prevented, preferably necro-haemorrhagic enteritis. The lactase can be administered to the bovine as a supplement such as a tablet, a powder, capsule or solution for oral use, or can be administered in a dairy-based milk replacer formulation.

An aspect of the invention relates to lactase for use in preventing an alimentary tract disorder caused by *Clostridium perfringens* in a bovine subject, wherein the lactase is orally administered to the bovine subject.

An aspect of the invention relates to lactase for use in preventing a *Clostridium perfringens* alpha-toxin related alimentary tract disorder in a bovine subject, wherein the lactase is orally administered to the bovine subject.

An aspect of the invention relates to lactase for use in increasing active immunity against an alimentary tract disorder in a bovine subject caused by *Clostridium perfringens,* wherein the lactase is orally administered to the bovine subject.

An aspect of the invention relates to lactase for use in increasing active immunity against a *Clostridium perfringens* alpha-toxin related alimentary tract disorder in a bovine subject, wherein the lactase is orally administered to the bovine subject.

An aspect of the invention relates to lactase for use in stimulating *Clostridium perfringens* alpha-toxin antibody production in a bovine subject, wherein the lactase is orally administered to the bovine subject.

An aspect of the invention relates to lactase for use in any one or more of: preventing an alimentary tract disorder caused by *Clostridium perfringens* in a bovine subject, preventing a *Clostridium perfringens* alpha-toxin related alimentary tract disorder in the bovine subject, increasing active immunity against an alimentary tract disorder in the bovine subject caused by *Clostridium perfringens,* increasing active immunity against a *Clostridium perfringens* alpha-toxin related alimentary tract disorder in the bovine subject and stimulating *Clostridium perfringens* alpha-toxin antibody production in the bovine subject, wherein the lactase is orally administered to the bovine subject.

An embodiment is the lactase for use according to the invention, wherein the lactase is provided in a dairy-based milk replacer formulation for bovines, preferably for cattle, more preferably for calves, wherein the dairy-based milk replacer formulation comprises at least 10 wt.% lactose based on dry matter, preferably at least 30 wt.% lactose based on dry matter, more preferably at least 40 wt.% lactose based on dry matter.

An aspect of the invention relates to use of lactase for preventing a bovine alimentary tract disorder caused by *Clostridium perfringens,* wherein the lactase is orally administered to the bovine subject.

An aspect of the invention relates to use of lactase for preventing a *Clostridium perfringens* alpha-toxin related alimentary tract disorder in a bovine subject, wherein the lactase is orally administered to the bovine subject.

An aspect of the invention relates to use of lactase for increasing active immunity against an alimentary tract disorder in a bovine subject caused by *Clostridium perfringens,* wherein the lactase is orally administered to the bovine subject.

An aspect of the invention relates to use of lactase for increasing active immunity against a *Clostridium perfringens* alpha-toxin related alimentary tract disorder in a bovine subject, wherein the lactase is orally administered to the bovine subject.

An aspect of the invention relates to use of lactase for stimulating *Clostridium perfringens* alpha-toxin antibody production in a bovine subject, wherein the lactase is orally administered to the bovine subject.

An aspect of the invention relates to use of lactase for any one or more of: preventing a bovine alimentary tract disorder caused by *Clostridium perfringens,* preventing a *Clostridium perfringens* alpha-toxin related alimentary tract disorder in the bovine subject, increasing active immunity against an alimentary tract disorder in the bovine subject caused by *Clostridium perfringens,* increasing active immunity against a *Clostridium perfringens* alpha-toxin related alimentary tract disorder in the bovine subject and stimulating *Clostridium perfringens* alpha-toxin antibody production in the bovine subject, wherein the lactase is orally administered to the bovine subject.

An aspect of the invention relates to a method for providing a dairy-based milk replacer formulation for bovines comprising lactase according to the invention, wherein the dairy-based milk replacer formulation comprising lactase comprises a lower lactose level than the dairy-based milk replacer formulation for bovines in which the lactase is omitted, comprising the steps of:
(a) providing a dairy-based milk replacer formulation for bovines comprising at least 20 wt.% lactose based on dry matter;
(b) providing lactase;
(c) mixing the dairy-based milk replacer formulation of step (a) with the lactase of step (b) and incubating for at least 5 minutes, preferably 5 minutes - 30 minutes, more preferably 10 minutes - 20 minutes, at a temperature of 20°C - 60°C, preferably 25°C - 40°C.

### Description

The present invention concerns lactase for use as a veterinary medicament, preferably as a medicament for a bovine, wherein the lactase is orally administered to the bovine subject.

The invention can also be worded as lactase for use in preventing a bovine alimentary tract disorder caused by *Clostridium perfringens,* wherein the lactase is orally administered to the bovine subject. An aspect of the invention relates to lactase for use in preventing an alimentary tract disorder caused by *Clostridium perfringens* in a bovine subject, wherein the lactase is orally administered to the bovine subject.

The invention can also be worded as lactase for use in preventing a *Clostridium perfringens* alpha-toxin related bovine alimentary tract disorder, wherein the lactase is orally administered to the bovine subject. An aspect of the invention relates to lactase for use in preventing a *Clostridium perfringens* alpha-toxin related alimentary tract disorder in a bovine subject, wherein the lactase is orally administered to the bovine subject.

Furthermore the invention can also be worded as lactase for use in increasing active immunity against a bovine alimentary tract disorder caused by *Clostridium perfringens,* wherein the lactase is orally administered to the bovine subject. An aspect of the invention relates to lactase for use in increasing active immunity against an alimentary tract disorder in a bovine subject caused by *Clostridium perfringens,* wherein the lactase is orally administered to the bovine subject.

The present invention also concerns lactase for use in increasing active immunity against a *Clostridium perfringens* alpha-toxin related bovine alimentary tract disorder, wherein the lactase is orally administered to the bovine subject. An aspect of the invention relates to lactase for use in increasing active immunity against a *Clostridium perfringens* alpha-toxin related alimentary tract disorder in a bovine subject, wherein the lactase is orally administered to the bovine subject.

The invention can also be worded as lactase for use in stimulating *Clostridium perfringens* alpha-toxin antibody production in bovines, wherein the lactase is orally administered to the bovine subject. An aspect of the invention relates to lactase for use in stimulating *Clostridium perfringens* alpha-toxin antibody production in a bovine subject, wherein the lactase is orally administered to the bovine subject.

In addition the invention can be worded as a method for preventing a bovine alimentary tract disorder caused by *Clostridium perfringens,* comprising orally administering lactase to a bovine subject. An aspect of the invention relates to use of lactase for preventing a bovine alimentary tract disorder caused by *Clostridium perfringens,* wherein the lactase is orally administered to the bovine subject.

The invention can also be worded as a method for preventing a *Clostridium perfringens* alpha-toxin related bovine alimentary tract disorder, comprising orally administering lactase to a bovine subject. An aspect of the invention relates to use of lactase for preventing a *Clostridium perfringens* alpha-toxin related alimentary tract disorder in a bovine subject, wherein the lactase is orally administered to the bovine subject.

Furthermore the invention can also be worded as a method for increasing active immunity against a bovine alimentary tract disorder caused by *Clostridium perfringens,* comprising orally administering lactase to a bovine subject. An aspect of the invention relates to use of lactase for increasing active immunity against an alimentary tract disorder in a bovine subject caused by *Clostridium perfringens,* wherein the lactase is orally administered to the bovine subject.

The present invention also concerns a method for increasing active immunity against a *Clostridium perfringens* alpha-toxin related bovine alimentary tract disorder, comprising orally administering lactase to a bovine subject. An aspect of the invention relates to use of lactase for increasing active immunity against a *Clostridium perfringens* alpha-toxin related alimentary tract disorder in a bovine subject, wherein the lactase is orally administered to the bovine subject.

The invention can also be worded as a method for stimulating *Clostridium perfringens* alpha-toxin antibody production in bovines comprising orally administering lactase to a bovine subject. An aspect of the invention relates to use of lactase for stimulating *Clostridium perfringens* alpha-toxin antibody production in a bovine subject, wherein the lactase is orally administered to the bovine subject.

Summarizing, the invention also concerns the use of lactase for preventing a bovine alimentary tract disorder caused by *Clostridium perfringens,* wherein the lactase is orally administered to the bovine subject, the use of lactase for preventing a *Clostridium perfringens* alpha-toxin related alimentary tract disorder in a bovine subject, wherein the lactase is orally administered to the bovine subject, the use of lactase for increasing active immunity against an alimentary tract disorder in a bovine subject caused by *Clostridium perfringens,* wherein the lactase is orally administered to the bovine subject, the use of lactase for increasing active immunity against a *Clostridium perfringens* alpha-toxin related alimentary tract disorder in a bovine subject, wherein the lactase is orally administered to the bovine subject and/or the use of lactase for stimulating *Clostridium perfringens* alpha-toxin antibody production in a bovine subject, wherein the lactase is orally administered to the bovine subject.

Furthermore, the invention concerns a method for providing a dairy-based milk replacer formulation for bovines comprising lactase, wherein the dairy-based milk replacer formulation comprising lactase comprises a lower lactose level than the dairy-based milk replacer formulation for bovines in which the lactase is omitted, comprising the steps of:
(a) providing a dairy-based milk replacer formulation for bovines comprising at least 20 wt.% lactose based on dry matter;
(b) providing lactase;
(c) mixing the dairy-based milk replacer formulation of step (a) with the lactase of step (b) and incubating for at least 5 minutes, preferably 5 minutes - 30 minutes, more preferably 10 minutes - 20 minutes, at a temperature of 20°C - 60°C, preferably 25°C - 40°C.

Furthermore, the invention concerns a method for providing a dairy-based milk replacer formulation for bovines comprising lactase, wherein the dairy-based milk replacer formulation comprising lactase comprises a lower lactose level than the dairy-based milk replacer formulation for bovines in which the lactase is omitted, comprising the steps of:
(a) providing a dairy-based milk replacer formulation for bovines comprising at least 20 wt.% lactose based on dry matter;
(b) providing lactase;
(c) mixing the dairy-based milk replacer formulation of step (a) with the lactase of step (b) and incubating for at least 5 minutes, preferably 5 minutes - 60 minutes, more preferably 10 minutes - 50 minutes, at a temperature of 20°C - 60°C, preferably 25°C - 45°C. For example, incubating is for 30 - 45 minutes such as 40 minutes, and for example, incubating is at a temperature of 38 - 44°C or 42°C±2°C.

Furthermore, the invention concerns the use of lactase, lactase for use, and/or a method comprising orally administering lactase, wherein the lactase is provided with a prescription to use the lactase in preventing an alimentary tract disorder caused by *Clostridium perfringens* in a bovine subject, in preventing a *Clostridium perfringens* alpha-toxin related alimentary tract disorder in a bovine subject, in increasing active immunity against an alimentary tract disorder in a bovine subject caused by *Clostridium perfringens,* in increasing active immunity against a *Clostridium perfringens* alpha-toxin related alimentary tract disorder in a bovine subject and/or in stimulating *Clostridium perfringens* alpha-toxin antibody production in a bovine subject and wherein the lactase is orally administered to a bovine subject.

In a preferred embodiment the lactase is administered separate from a lactose comprising diet and/or is not provided in a kit of parts comprising a first container comprising a high-lactose dairy-based milk replacer for bovines in dry form such as powder form and a second container comprising lactase.

It is noted that wherever in the present description wording like "use of lactase comprising orally administering lactase to a bovine subject", "lactase for use comprising orally administering lactase to a bovine subject" or "a method comprising orally administering lactase to a bovine subject" is used, these wordings all refer to the methods and uses according to the present invention.

### Lactase

Lactase is an enzyme known in the art and commercially provided by several suppliers. Lactase is a glycoside hydrolase which hydrolyses the disaccharide lactose into constituent galactose and glucose monomers. Lactase is able to hydrolyse lactose both at neutral pH conditions such as in milk or a milk replacer as under acid conditions such as in-stomach and in the intestinal tract. The activity of lactase is expressed in U and can be determined according to Jasewicz et al. (Quantitative determination of lactase. J. Dairy Sci. 44:393-400, 1961). A unit U of lactase is defined as the amount of enzyme which will liberate 1 µM of glucose from lactose in 15 min. at 37°C. Preferably the lactase activity is measured at pH 7. To reach a specific lactose conversion, a specific amount of lactase (or lactase encompassing a specific amount of lactase activity) should be provided for a specific time at a specific temperature. Suppliers of lactase provide this information, and next to this the amount, temperature and time to reach a desired lactose conversion can easily be determined by routine experimentation. The lactase of the lactase-enriched dairy-based milk replacer is active, so not denatured lactase. Active with regards to lactase has the meaning that the lactase when brought into contact with dissolved lactose under typical lactose-split reaction conditions will hydrolyse lactose.

In a preferred embodiment the lactase is provided as a single dosage form for oral application, preferably as a tablet, powder, capsule or solution for oral use. Preferably the single dosage form is a supplement.

In a preferred embodiment the lactase is active between pH 4 - 7, more preferably between pH 5 - 7, most preferably between 5.5 - 7. The pH of a dairy-based milk replacer is about 6, so at this pH range lactase when present in the dairy-based milk replacer will break down the lactase of a dairy-based milk replacer. Moreover when a bovine is being fed a dairy-based milk replacer the dairy-based milk replacer will enter the abomasum of the bovine. Upon the intake of the dairy-based milk replacer the pH in the abomasum will rise to the pH of this milk replacer and will remain at this pH for several hours. Lactase being fed to the bovine that is active between pH 4-7 will therefore be active in the abomasum and break down lactose present in the abomasum.

### Lactose comprising diet

In one aspect of the invention lactase is administered to a bovine subject who is fed a lactose comprising diet. A diet concerns the nutrition the bovine usually receives, so concerns the total of the meals eaten by that bovine. Although in some case a diet is fed ad libitum, typically the diet of bovines comprises several meals. These meals may have the same composition. A lactose comprising diet is a diet that comprises lactose. Preferably, the lactose comprising diet comprises at least 10 wt.% lactose on total dry weight of the diet, more preferably at least 20 wt.%, even more preferably at least 30 wt.%, most preferably at least 40 wt.%. Preferably, the lactose comprising diet comprises 10 - 60 wt.% lactose on total dry weight of the diet, more preferably 20 - 50 wt.%, even more preferably 30 - 45 wt.%.

Preferably, the lactose comprising diet is dairy based. In a preferred embodiment the lactose comprising diet is selected from any one of: a milk, preferably bovine milk and a milk replacer for bovines, preferably a dairy-based milk replacer for bovines. In a preferred embodiment the lactose comprising diet comprises at least one lactose comprising meal. For this embodiment preferably the lactose comprising meal comprises at least 10 wt.% lactose on total dry weight of the diet, more preferably at least 20 wt.%, even more preferably at least 30 wt.%, most preferably at least 40 wt.%. Preferably the lactose comprising meal of this embodiment comprises 10 - 60 wt.% lactose on total dry weight of the diet, more preferably 20 - 50 wt.%, even more 30 - 45 wt.%. Preferably the lactose comprising meal of this embodiment is selected from any one of: a milk, preferably bovine milk, and a milk replacer for bovines, preferably a dairy-based milk replacer for bovines.

In a preferred embodiment the lactose comprising diet has not been treated with transglucosidase and/or does not comprise transglucosidase. For this embodiment preferably the transglucosidase not comprised in the lactose comprising diet concerns both active and inactivated transglucosidase.

Preferably, the lactase is administered to the bovine subject before, during or after the bovine subject is fed the lactose-comprising diet, preferably 5 seconds - 5 minutes before, during or 5 seconds - 5 minutes after the bovine subject is fed with the lactose-comprising diet, more preferably during the bovine subject is fed with the lactose-comprising diet. Preferably the lactose comprising diet comprises at least one lactose comprising meal and the lactase is administered to the bovine subject before, during or after the bovine subject is fed the lactose-comprising meal, preferably 5 seconds - 5 minutes before, during or 5 seconds - 5 minutes after the bovine subject is fed with the lactose-comprising meal, more preferably during the bovine subject is fed with the lactose-comprising meal.

Preferably, lactase is provided in an amount of 100 - 4000 U lactase per day, more preferably an amount of 200 - 2000 U lactase per day, even more preferably an amount of 400 - 1500 U lactase per day, most preferably an amount of 700 - 1000 U lactase per day. Preferably lactase is provided in an amount of at least 200 U lactase per kg lactose in the daily diet, more preferably at least 800 U lactase per kg lactose in the daily diet, even more preferably at least 1500 U lactase per kg lactose in the daily diet, most preferably at least 2000 U lactase per kg lactose in the daily diet. More preferably lactase is provided in an amount of at least 6000 U lactase per kg lactose in the daily diet, more preferably at least 12000 U lactase per kg lactose in the daily diet, even more preferably at least 30000 U lactase per kg lactose in the daily diet, most preferably at least 35000 U lactase per kg lactose in the daily diet. Preferred for the lactase for use according to the invention, lactase is provided in an amount of 100 - 2000 U lactase per day, preferably an amount of 400 - 1000 U lactase per day. More preferred, for the lactase for use according to the invention, the lactase is provided in an amount of 2000 - 35000 U lactase per day, preferably an amount of 4000 - 30000 U lactase per day.

### Dairy based milk replacer formulation for bovines

The lactase may be provided in a dairy based milk replacer formulation for bovines. Preferably the dairy based milk replacer formulation comprises 50 wt.% or more of dairy protein based on total protein, more preferably 70 wt.% or more, even more preferably 90 wt.% or more. In addition or alternatively, the dairy based milk replacer formulation preferably comprises 30 wt.% or more of dry weight of dairy-based ingredients based on total dry weight of the formulation, more preferably 50 wt.% or more, even more preferably 60 wt.% or more. Glucose and galactose that are obtained from lactose by enzymatic hydrolysis, e.g. lactase hydrolysis, are dairy-based ingredients. The dairy-based ingredients are from mammalian milk, such as bovine milk. Preferably, the dairy-based ingredients are from one or more selected from the list consisting of cow milk, goat milk and sheep milk. Preferably the dairy based milk replacer formulation is cow milk-based, goat milk-based, sheep milk-based or a combination thereof, more preferably the dairy based milk replacer formulation is a cow milk-based formulation.

Dairy proteins are typically more digestible than non-dairy proteins and provide a desirable amino acid profile for calf growth. Therefore, preferably, dairy products that provide dairy protein are used in the manufacturing of milk replacers. These include whey, whey protein concentrate, whey solubles, skim milk and casein. Whey products are utilized as important protein sources in dairy-based milk replacers. In one embodiment the dairy based milk replacer formulation preferably comprises 40 wt.% or more of whey protein based on total protein, more preferably 60 wt.% or more, even more preferably 80 wt.% or more. Casein products are also utilized as important protein sources in dairy-based milk replacers. In one embodiment the dairy based milk replacer formulation preferably comprises 40 wt.% or more of casein based on total protein, more preferably 60 wt.% or more, even more preferably 80 wt.% or more. It is preferred that the lactase is provided in a dairy-based milk replacer formulation for bovines, preferably for cattle, more preferably for calves, wherein the dairy-based milk replacer formulation comprises at least 10 wt.% lactose based on dry matter, preferably at least 30 wt.% lactose based on dry matter, more preferably at least 40 wt.% lactose based on dry matter.

The amounts of macro-nutrients in a dairy based milk replacer formulation are tailored to optimize growth and well-being of the bovine, such as a calf. Preferably, the dairy based milk replacer formulation comprises 10 - 35 wt.% protein based on total dry weight of the formulation, 10 - 30 wt.% lipids based on total dry weight of the formulation and 30 - 60 wt.% of digestible carbohydrates based on total dry weight of the formulation, more preferably comprise 17-28 wt.% protein based on total dry weight of the formulation, 15 - 25 wt.% lipids based on total dry weight of the formulation and 35 - 55 wt.% of digestible carbohydrates based on total dry weight of the formulation, most preferably comprise 20 - 25 wt.% protein based on total dry weight of the formulation, 20 - 25 wt.% lipids based on total dry weight of the formulation and 40 - 50 wt.% of digestible carbohydrates based on total dry weight of the formulation.

Preferably, the dairy-based milk replacer formulation for bovines comprises at least 10 wt.% lactose on total dry weight of the formulation, more preferably at least 20 wt.%, even more preferably at least 30 wt.%, most preferably at least 40 wt.%. Preferably the dairy-based milk replacer formulation for bovines comprises 10 - 60 wt.% lactose on total dry weight of the formulation, more preferably 20 - 55 wt.%, even more preferably 30 - 50 wt.%, most preferably 40 - 47 wt.%.

The dairy based milk replacer formulation may be liquid or solid. In one embodiment the dairy based milk replacer formulation preferably is liquid. Preferably in this embodiment the dry matter content is 5 - 30 wt.% based on total weight of the dairy based milk replacer formulation , more preferably 10 - 20 wt.%, even more preferably 12 - 17 wt.%, most preferably 12.5 -15 wt.%. In another preferable embodiment the the dairy based milk replacer formulation is solid, for example a powder, a granulate or a pellet, preferably a powder. Thus, preferably, the dairy-based milk replacer formulation for bovines is a liquid, a powder, a granulate or a pellet, preferably a liquid.

The dairy based milk replacer formulation when in liquid form may be defined by its osmolality. Osmolality is expressed in osmoles per liter, abbreviated as osmol/L. A high osmolality may induce diarrhea in bovines. Preferably, the osmolality of the liquid dairy based milk replacer formulation is 200 - 800 osmol/L, more preferably 300 - 700 osmol/L, most preferably 300 - 500 osmol/L.

In the embodiment the lactase is provided in a dairy-based milk replacer formulation for bovines, preferably lactase is provided in an amount of 100 U or more lactase per kg dry weight of the dairy-based milk replacer formulation, preferably 400 U or more, more preferably 700 U or more, even more preferably 1000 U or more. Also preferred is when the lactase for use according to the invention is provided in an amount of 4000 U or more lactase per kg dry weight of the dairy-based milk replacer formulation, preferably 8000 U or more, most preferably 12000 U or more, such as 10000 - 16000 U lactase per kg dry weight of the dairy-based milk replacer formulation. Preferably, lactase is provided in an amount of 200 U or more lactase per kg lactose of the dairy-based milk replacer formulation, more preferably 800 U or more, even more preferably 1500 U or more, most preferably 2000 U or more. Also preferred is when the lactase is provided in an amount of 6000 U or more lactase per kg lactose of the dairy-based milk replacer formulation, more preferably 12000 U or more, even more preferably 18000 U or more, most preferably 24000 U or more, such as 20000 - 35000 U.

In some instance part of the carbohydrates of the dairy-based milk replacer formulation for bovines comprises carbohydrates other than lactose. Preferably the dairy-based milk replacer formulation for bovines comprises carbohydrates other than lactose, more preferably the dairy-based milk replacer formulation for bovines comprises one or more carbohydrates selected from the list consisting of glucose, fructose, galactose, ribose, mannose, arabinose, xylose, dextrose, sucrose, trehalose, maltose, iso-maltulose, galacto-oligosaccharides (GOS), fructo-oligosaccharides (FOS), xylo-oligosaccharides (XOS), galacturonic-oligosacharides, isomalto-oligosaccharides, raffinose, maltodextrins, and starch. Preferably the concentration of the sum of these carbohydrates is 10 - 40 wt.% on dry matter of the low-lactose dairy-based milk replacer, more preferably 15 - 30 wt.%.

In one embodiment the dairy-based milk replacer formulation comprises 20 - 50 wt.% lactose based on total dry weight of the milk replacer formulation and 10 - 40 wt.% other carbohydrates based on total dry weight of the milk replacer, preferably 20 - 40 wt.% lactose and 20 - 40 wt.% other carbohydrates; wherein the other carbohydrates preferably are selected from the list consisting of glucose, fructose, galactose, ribose, mannose, arabinose, xylose, dextrose, sucrose, trehalose, maltose, iso-maltulose, galacto-oligosaccharides (GOS), fructo-oligosaccharides (FOS), xylo-oligosaccharides (XOS), galacturonic-oligosaccharides, isomalto-oligosaccharides, raffinose, maltodextrins, and starch.

Milk replacers are routinely prepared at the farm by mixing the milk replacer formulation in powder form with a liquid, and typically with water. As known in the art, the optimal feeding temperature for bovine calves is 39°C so the mix is commonly prepared to reach this temperature and typically a final dry matter of 12.5 wt.% - 15 wt.% on total weight is aimed for. Moreover this temperature is known in the art at which the activity of lactase is high. In a preferred embodiment the dairy-based milk replacer formulation is fed to the bovine subject after a waiting time of 5 minutes or more after providing the lactase to the dairy-based milk replacer formulation, preferably 5 minutes - 15 minutes, more preferably 10 minutes - 50 minutes wherein the dairy-based milk replacer formulation during the waiting time has a temperature of 20°C - 60°C, preferably 30°C - 50°C; and wherein in case the dairy-based milk replacer formulation is a powder, a granulate or a pellet, the powder, a granulate or a pellet has been dissolved before the waiting time.

### Application

The invention is directed towards a *bovine* subject and in preferred embodiments also concerns a diet for *bovines* and/or a dairy-based milk replacer formulation for *bovines.* Bovines are domestic animals of the species *Bos Taurus*, *Bubalus bubalis*, *Bison spp.* or hybrids thereof. Preferably the bovine is cattle. Cattle are animals of the species *Bos Taurus.*

More preferably the bovine is a calf, even more preferably a veal calf, a beef calf and/or a dairy calf, most preferably the bovine is a veal calf. Veal calves are calves raised for veal, beef calves are calves raised for beef, dairy calves are calves raised to produce milk. A calf is a cattle with an age of 0 to 8 months. Wherever in this description the term "calf" is used, this can be replaced by "cattle with an age of 0 to 8 months". Preferred is the lactase for use according to the invention, wherein the bovine subject is a cattle subject, preferably wherein the bovine subject is a calf, even more preferably wherein the bovine subject is a veal calf, a beef calf or a dairy calf, most preferably wherein the bovine subject is a veal calf.

A new-born bovine receives passive immunity towards *C*. *perfringens* from the colostrum of its mother, more in particular, passive immunity towards alpha toxin such as alpha toxin from *C*. *perfringens.* As colostrum is only provided just after birth the effect is not lasting, although maternal antibodies have been identified up to a few months after birth. In general this passive immunity dwindles the first few weeks of life and the new-born bovine needs to build up active immunity itself to remain protected from *C*. *perfringens* infections and related consequences thereof such as detrimental alpha toxin levels. Active immunity refers to the process of exposing the body to an antigen to generate an adaptive immune response: the response takes days/weeks to develop but may be long lasting - even lifelong. The general mechanism to build up active immunity towards *C*. *perfringens* is related to the presence of a low amount of *C*. *perfringens* in the intestines, which produces a low amount of toxins towards which the new-born bovine subsequently develops active immunity. The presence of *C*. *perfringens* alpha toxin antibodies in the serum of the bovine is a clear indicator for immunity against *C*. *perfringens.* The mechanism for building active immunity seems hampered for bovines receiving relative high amounts of lactose such as for example present in conventional milk replacers. This is visible in that the serum amount also referred to as 'titer' of alpha toxin anti-bodies decreases in time for these bovines.

Considering that active immunity against *C. perfringens* is best build up early in life, for example to early raise (improved) protection against alpha-toxin related disorders, in particular necro-haemorrhagic enteritis and related death, preferably the lactase of the invention is best provided early in life. Preferably, the lactase is administered to bovines starting from 28 days after birth of said bovine, more preferably starting from 14 day after birth, even more preferably starting from 7 day after birth, even more preferably starting from the first feeding after colostrum feeding has stopped, most preferably directly after birth. In addition, considering that the that active immunity against *C*. *perfringens* may be best build up at the moment the passive immunity decreases, the lactase is administered to bovines starting from at least 1 day after birth of said bovine, preferably starting from at least 7 days after birth of said bovine, more preferably starting from at least 14 day after birth, even more preferably starting from at least 28 days after birth, even more preferably starting from at least 42 days after birth, such as starting from any of at least 1, 2, 3, 4, 5, 6, 7, 14, 28, 42, 100, 120, 150, 200, 224, 250, 300 days after birth. Preferably the lactase is administered to bovines from 1 days to 300 days after birth of said bovine, preferably from 7 days to 250 days after birth of said bovine, more preferably from 14 day to 200 days after birth, even more preferably from 28 days to 150 after birth, even more preferably from 42 days to 60 days after birth, wherein the lactase is consecutively administered for 20 days or more within these periods.

To maximize the impact of the lactase of the invention, so to maximize the build-up of active immunity (e.g. build-up of anti-alpha toxin antibody titer), the lactase preferably is administered for a prolonged time. Preferably the lactase is consecutively administered for 20 days or more, preferably for 40 days or more, even more preferably for 60 days or more, most preferably for 80 days or more. In case the lactase is provided in a dairy-based milk replacer formulation for bovines, preferably the dairy-based milk replacer formulation is administered to bovines starting from 14 days after birth of said bovine, more preferably starting from 7 day after birth, even more preferably starting from the first feeding after colostrum feeding has stopped, most preferably directly after birth. Preferably in case the lactase is provided in a dairy-based milk replacer formulation for bovines, the dairy-based milk replacer formulation is consecutively administered for 20 days or more, more preferably for 40 days or more, even more preferably for 60 days or more.

Bovines are susceptible for *C*. *perfringens* related alimentary tract disorders. Several alimentary tract disorders are known that are directly related to insufficient immunity against *C*. *perfringens,* and a low serum *C*. *perfringens* alpha toxin antibody amount is indicative for insufficient immunity. These *C*. *perfringens* related alimentary tract disorders are prevented by increasing active immunity against *C*. *perfringens,* for which an increased serum alpha toxin antibody amount is indicative.

Therefore, in one embodiment the invention concerns lactase for use in preventing a bovine alimentary tract disorder caused by *Clostridium perfringens.*

Consequently in another embodiment the invention may also be phrased as lactase for use in preventing a *Clostridium perfringens* alpha-toxin related bovine alimentary tract disorder.

In another embodiment the invention can be phrased as a lactase for use in increasing active immunity against a bovine alimentary tract disorder caused by *Clostridium perfringens.*

In yet another embodiment the invention can be phrased as lactase for use in increasing active immunity against a *Clostridium perfringens* alpha-toxin related bovine alimentary tract disorder.

In a further embodiment the invention concerns lactase for use in stimulating *Clostridium perfringens* alpha-toxin antibody production in bovines.

Thus, preferred is the lactose for use according to the invention, wherein the active immunity against a bovine alimentary tract disorder caused by *Clostridium perfringens* is increased compared to a bovine subject that is not administered the lactase, the active immunity against a *Clostridium perfringens* alpha-toxin related bovine alimentary tract disorder is increased compared to a bovine subject that is not administered the lactase and/or the *Clostridium perfringens* alpha-toxin antibody production in bovines is stimulated compared to a bovine subject that is not administered the lactase.

The lactase for use preferably is administered to a bovine subject who is fed a lactose comprising diet. Preferred is the use of lactase of the invention, wherein the lactose-comprising diet is selected from any one of: a milk, preferably bovine milk, a milk replacer for bovines, preferably a dairy-based milk replacer for bovines. Preferably the lactase for use is provided in a dairy-based milk replacer formulation for bovines. Preferred is the use of lactase according to the invention, wherein the lactase is provided as a dairy-based milk replacer formulation for bovines comprising at least 10 wt.% lactose based on dry matter, preferably at least 30 wt.% lactose based on dry matter, more preferably at least 40 wt.% lactose based on dry matter. Typically, the use of the lactase according to the invention is the use wherein the bovine subject is a cattle subject, preferably wherein the bovine subject is a calf, even more preferably wherein the bovine subject is a veal calf, a beef calf or a dairy calf, most preferably wherein the bovine subject is a veal calf.

Bovine alimentary tract disorder related *Clostridium perfringens* are enterotoxaemia, necro-haemorrhagic enteritis, clostridial abomasitis, haemorrhagic enteritis, haemorrhagic abomasitis and abomasal ulceration. Necro-haemorrhagic enteritis is often called enterotoxaemia. Both conditions relate to *C*. *perfringens,* however enterotoxaemia is applied to a wider ranges of diseases caused by *C*. *perfringens,* whilst necro-haemorrhagic enteritis only applies for the disease caused by *C*. *perfringens* type A and which is characterized by intestinal necrosis and haemorrhages. In an embodiment of the invention the lactase is for use for preventing of or inducing active immunity against enterotoxaemia, necro-haemorrhagic enteritis, clostridial abomasitis, haemorrhagic enteritis, haemorrhagic abomasitis and abomasal ulceration, preferably is necro-haemorrhagic enteritis and/or enterotoxaemia, more preferably is necro-haemorrhagic enteritis. Thus, preferred is the lactase for use according to the invention, wherein the *Clostridium perfringens* alpha-toxin related bovine alimentary tract disorder or the bovine alimentary tract disorder caused by *Clostridium perfringens* is any one or more selected from the list consisting of enterotoxaemia, necro-haemorrhagic enteritis, clostridial abomasitis, haemorrhagic enteritis, haemorrhagic abomasitis and abomasal ulceration, preferably the *Clostridium perfringens* alpha-toxin related bovine alimentary tract disorder or the bovine alimentary tract disorder caused by *Clostridium perfringens* is necro-haemorrhagic enteritis and/or enterotoxaemia, more preferably necro-haemorrhagic enteritis.

In one embodiment of the invention *Clostridium perfringens* preferably is selected from one or more from the group consisting of *Clostridium perfringens* type A, *Clostridium perfringens* type B, *Clostridium perfringens* type C, *Clostridium perfringens* type D, *Clostridium perfringens* type E, more preferably the *Clostridium perfringens* is *Clostridium perfringens* type A. All *Clostridium perfringens* types are capable to produce alpha-toxin, and therefore to induce alpha-toxin related conditions. However especially *Clostridium perfringens* type A is known to relate to bovine alimentary tract disorder caused by *Clostridium perfringens* and especially to *Clostridium perfringens* alpha-toxin related bovine alimentary tract disorder, particularly to necro-haemorrhagic enteritis.

For necro-haemorrhagic enteritis and other alimentary tract disorders caused by *Clostridium perfringens* a second toxin may be involved; perfringolysin O. Exposure of the young bovine to low amounts of *Clostridium perfringens* and consequently to the toxins of *Clostridium perfringens* may furthermore, so next to inducing active immunity against *Clostridium perfringens* alpha-toxin, induce active immunity against *Clostridium perfringens* perfringolysin O. In one embodiment, preferably the lactose for use is furthermore for use in stimulating *Clostridium perfringens* perfringolysin O antibody production in bovines. Preferably, the lactose for use is furthermore for use in increasing active immunity against a *Clostridium perfringens* perfringolysin O related bovine alimentary tract disorder. More preferably the lactose for use is furthermore for use in stimulating *Clostridium perfringens* alpha toxin and *Clostridium perfringens* perfringolysin O antibody production in bovines. More preferably the lactose for use is furthermore for use in increasing active immunity against a *Clostridium perfringens* perfringolysin O and *Clostridium perfringens* alpha toxin related bovine alimentary tract disorder.

In the context of the present invention, 'prevention' of a disease or certain disorder also means 'reduction of the risk' of a disease or certain disorder and also means 'treatment of a bovine at risk' of said disease or said certain disorder.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The embodiments of the invention described herein can operate in combination and cooperation, unless specified otherwise.

### Description of the drawings

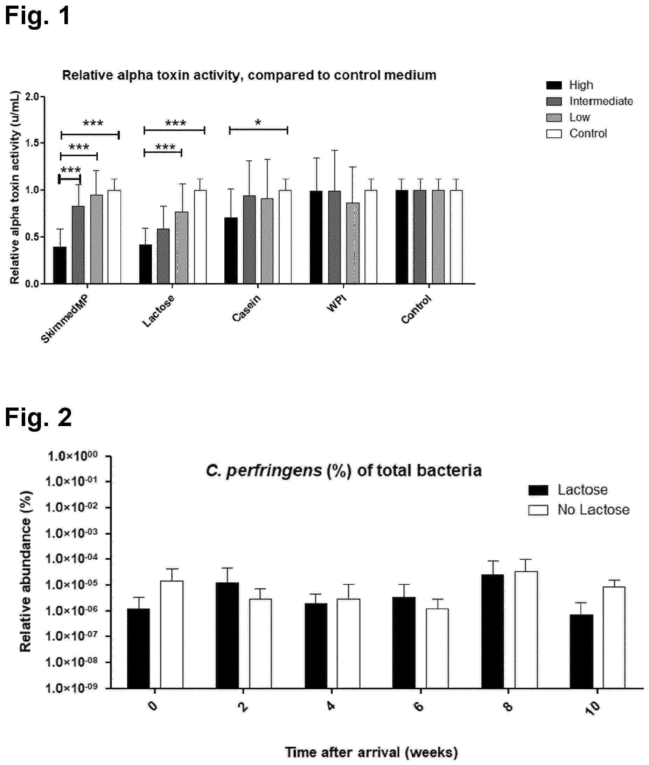

Figure 1 depicts the relative alpha toxin activity of BCP20 when supplemented with skimmed milk powder and its subcomponents lactose, casein and whey protein isolate (WPI) at different levels of concentration: high (approximately as fed), intermediate (10-fold dilution) and low (100-fold dilution). Values are expressed as fraction of the control, which is adjusted to 1.00 U/mL.
Figure 2 depicts the relative presence of *C*. *perfringens* in faecal samples throughout the study, for calves that received a normal milk replacer (non-lactase treated) or a lactase-treated milk replacer diet. Values are calculated by the ratio of absolute numbers of *C*. *perfringens* to total bacteria, which were determined using qPCR. The results were then log-transformed.

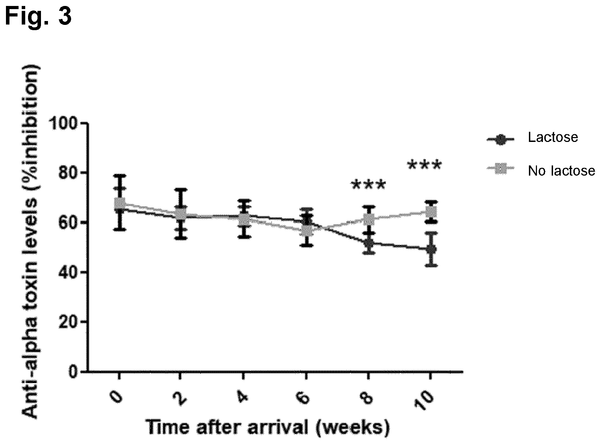

Figure 3 depicts the production of antibody against alpha toxin throughout time. The anti-alpha toxin levels (% inhibition) is determined by a blocking ELISA test (K291, Bio-X, Belgium). A higher anti-alpha toxin level corresponds to a higher serum level of anti alpha-toxin antibodies in the blood of the calf. Calves that received lactose, start at the same concentration (anti-alpha toxin level) as the group that was administered lactase, but the concentrations decrease (less alpha-toxin is inhibited), whereas the antibody levels (% inhibition) of the 'no lactose' calves remain high (lactose = black dots, no lactose = grey squares).

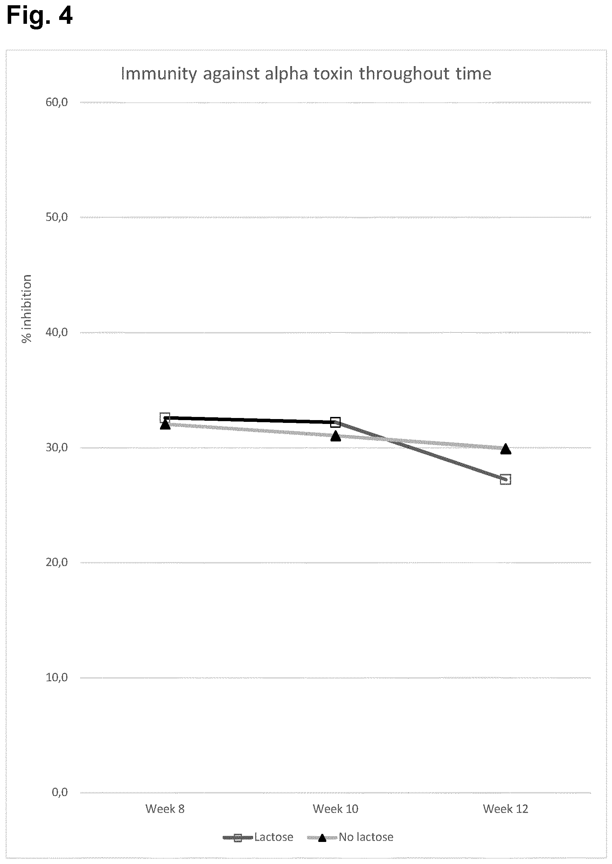

Figure 4 depicts the production of antibody against alpha toxin throughout time for two groups of 40 bovine calves. The anti-alpha toxin (antibody) levels (% inhibition) is determined by a blocking ELISA test (K291, Bio-X, Belgium). A higher anti-alpha toxin level corresponds to a higher serum level of anti alpha-toxin antibodies in the blood of the calve. Calves that received lactose, start at the same concentration (anti-alpha toxin level ) as the 'no lactose' group, but the concentrations decrease (less alpha-toxin is inhibited; open squares in the figure, 'Lactose') after 12 weeks from the start of feeding milk replacer, whereas the antibody levels (% inhibition) of the 'no lactose' calves remain high (lactose = open squares, no lactose = black triangles).

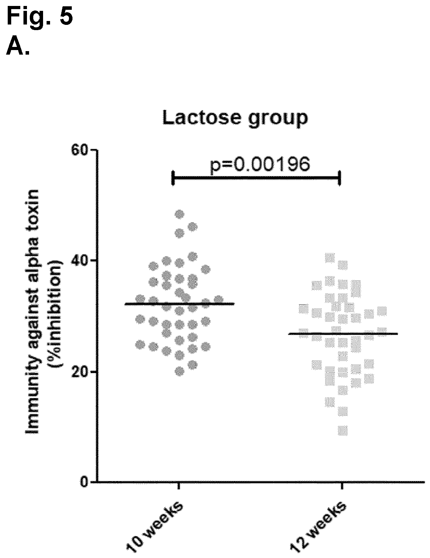

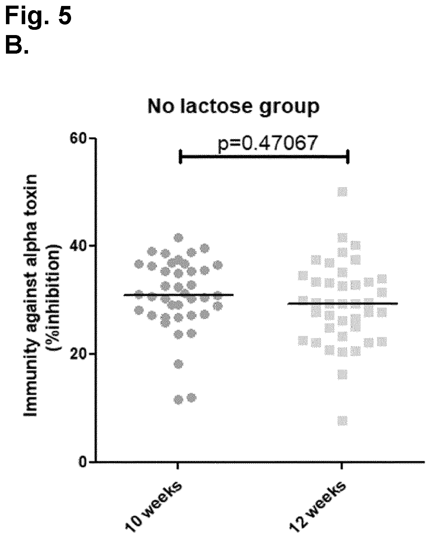

Figure 5A-C depict the anti-alpha toxin antibody titers (expressed as immunity against alpha-toxin in % inhibition) at week 10 and at week 12 for the group of calves which received the high-lactose milk replacer (Figure 5A), and for the group of calves which received the milk replacer without lactose (Figure 5B), and depict the comparison between the immunity against alpha-toxin (% inhibition) at week 12 for the group of calves fed with milk replacer comprising the regular amount of lactose ('lactose') and the group of calves fed with milk replacer treated with lactase before being fed to the calves ('No lactose').

### List of embodiments

1. Lactase for use in any one or more of: preventing an alimentary tract disorder caused by *Clostridium perfringens* in a bovine subject, preventing a *Clostridium perfringens* alpha-toxin related alimentary tract disorder in the bovine subject, increasing active immunity against an alimentary tract disorder in the bovine subject caused by *Clostridium perfringens,* increasing active immunity against a *Clostridium perfringens* alpha-toxin related alimentary tract disorder in the bovine subject and stimulating *Clostridium perfringens* alpha-toxin antibody production in the bovine subject, wherein the lactase is orally administered to the bovine subject.
2. Lactase for use according to embodiment 1, wherein the *Clostridium perfringens* alpha-toxin related bovine alimentary tract disorder or the bovine alimentary tract disorder caused by *Clostridium perfringens* is any one or more selected from the list consisting of enterotoxaemia, necro-haemorrhagic enteritis, clostridial abomasitis, haemorrhagic enteritis, haemorrhagic abomasitis and abomasal ulceration, preferably the *Clostridium perfringens* alpha-toxin related bovine alimentary tract disorder or the bovine alimentary tract disorder caused by *Clostridium perfringens* is necro-haemorrhagic enteritis and/or enterotoxaemia, more preferably necro-haemorrhagic enteritis.
3. Lactase for use according to embodiment 1 or 2, wherein the lactase is provided as a single dosage form for oral application, preferably as a tablet, powder, capsule or solution for oral use.
4. Lactase for use according to any one of the embodiments 1-3, wherein the lactase is administered to a bovine subject who is fed a lactose-comprising diet, and wherein optionally the lactase is administered to the bovine subject before, during or after the bovine subject is fed the lactose-comprising diet, preferably 5 seconds - 5 minutes before, during or 5 seconds - 5 minutes after the bovine subject is fed with the lactose-comprising diet, more preferably during the bovine subject is fed with the lactose-comprising diet, and/or wherein optionally the lactose-comprising diet is selected from any one of: a milk, preferably bovine milk and a milk replacer for bovines, preferably a dairy-based milk replacer for bovines.
5. Lactase for use according to embodiment 4, wherein the lactose-comprising diet comprises at least 10 wt.% lactose based on dry matter, preferably at least 30 wt.% lactose based on dry matter, more preferably at least 40 wt.% lactose based on dry matter.
6. Lactase for use according to any one of the embodiments 1-3, wherein the lactase is provided in a dairy-based milk replacer formulation for bovines, preferably for cattle, more preferably for calves, wherein the dairy-based milk replacer formulation comprises at least 10 wt.% lactose based on dry matter, preferably at least 30 wt.% lactose based on dry matter, more preferably at least 40 wt.% lactose based on dry matter, and wherein optionally the dairy-based milk replacer formulation for bovines is a liquid, a powder, a granulate or a pellet, preferably a liquid.
7. Lactase for use according to embodiment 6, wherein the dairy-based milk replacer formulation is fed to the bovine subject after a waiting time of 5 minutes or more after providing the lactase to the dairy-based milk replacer formulation, preferably 5 - 15 minutes wherein the dairy-based milk replacer formulation during the waiting time has a temperature of 20 - 60°C; and wherein in case the dairy-based milk replacer formulation is a powder, a granulate or a pellet, the powder, granulate or pellet has been dissolved before the waiting time.
8. Lactase for use according to embodiment 6 or 7, wherein dairy-based milk replacer formulation comprises 50 wt.% or more of dairy protein based on total protein, preferably 70 wt.% or more.
9. Lactase for use according to any one of the embodiments 6-8, wherein the dairy-based milk replacer formulation comprises 10 - 35 wt.% protein based on total dry weight of the milk replacer, 10 - 30 wt.% lipids based on total dry weight of the milk replacer formulation and 30 - 60 wt.% of digestible carbohydrates based on total dry weight of the milk replacer formulation.
10. Lactase for use according to any one of the embodiments 6-9, wherein the dairy-based milk replacer formulation comprises 10 - 60 wt.% lactose based on dry matter, preferably 20 - 55 wt.%, more preferably 30 - 50 wt.%.
11. Lactase for use according to any one of the embodiments 6-10, wherein lactase is provided in an amount of 100 U or more lactase per kg dry weight of the dairy-based milk replacer formulation, preferably 400 U or more, most preferably 700 U or more, or wherein lactase is provided in an amount of 4000 U or more lactase per kg dry weight of the dairy-based milk replacer formulation, preferably 8000 U or more, most preferably 12000 U or more.
12. Lactase for use according to any one of the previous embodiments, wherein the lactase is administered to the bovine subject starting from 28 days after birth of said bovine subject, preferably starting from 14 day after birth, even more preferably starting from 7 day after birth most preferably starting directly after birth, and/or wherein the lactase is consecutively administered for 20 days or more, preferably for 40 days or more, more preferably for 60 days or more.
13. Lactase for use according to any one of the preceding embodiments, wherein the bovine subject is a cattle subject, preferably wherein the bovine subject is a calf, even more preferably wherein the bovine subject is a veal calf, a beef calf or a dairy calf, most preferably wherein the bovine subject is a veal calf.
14. Lactase for use according to any one of the preceding embodiments, wherein lactase is provided in an amount of 100 - 2000 U lactase per day, preferably an amount of 400 - 1000 U lactase per day, or wherein lactase is provided in an amount of 2000 - 35000 U lactase per day, preferably an amount of 4000 - 30000 U lactase per day.
15. Lactase for use according to any one of the preceding embodiments, wherein the active immunity against a bovine alimentary tract disorder caused by *Clostridium perfringens* is increased compared to a bovine subject that is not administered the lactase, the active immunity against a *Clostridium perfringens* alpha-toxin related bovine alimentary tract disorder is increased compared to a bovine subject that is not administered the lactase and/or the *Clostridium perfringens* alpha-toxin antibody production in bovines is stimulated compared to a bovine subject that is not administered the lactase.
16. Use of lactase for any one or more of: preventing a bovine alimentary tract disorder caused by *Clostridium perfringens,* preventing a *Clostridium perfringens* alpha-toxin related alimentary tract disorder in the bovine subject, increasing active immunity against an alimentary tract disorder in the bovine subject caused by *Clostridium perfringens,* increasing active immunity against a *Clostridium perfringens* alpha-toxin related alimentary tract disorder in the bovine subject, and stimulating *Clostridium perfringens* alpha-toxin antibody production in the bovine subject, wherein the lactase is orally administered to the bovine subject.
17. Use of lactase according to embodiment 16, wherein the lactase is administered to a bovine subject who is fed a lactose-comprising diet, wherein optionally the lactose-comprising diet is selected from any one of: a milk, preferably bovine milk, a milk replacer for bovines, preferably a dairy-based milk replacer for bovines.
18. Use of lactase according to embodiment 16 or 17, wherein the lactase is provided as a dairy-based milk replacer formulation for bovines comprising at least 10 wt.% lactose based on dry matter, preferably at least 30 wt.% lactose based on dry matter, more preferably at least 40 wt.% lactose based on dry matter.
19. Use of lactase according to any one of the embodiments 16-18, wherein the bovine subject is a cattle subject, preferably wherein the bovine subject is a calf, even more preferably wherein the bovine subject is a veal calf, a beef calf or a dairy calf, most preferably wherein the bovine subject is a veal calf.
20. Method for providing a dairy-based milk replacer formulation for bovines comprising lactase according to any one of the embodiments 6-11, wherein the dairy-based milk replacer formulation comprising lactase comprises a lower lactose level than the dairy-based milk replacer formulation for bovines in which the lactase is omitted, comprising the steps of:
   (a) providing a dairy-based milk replacer formulation for bovines comprising at least 20 wt.% lactose based on dry matter;
   (b) providing lactase;
   (c) mixing the dairy-based milk replacer formulation of step (a) with the lactase of step (b) and incubating for at least 5 minutes, preferably 5 minutes - 30 minutes, more preferably 10 minutes - 20 minutes, at a temperature of 20°C - 60°C, preferably 25°C - 40°C.
21. Method for providing a dairy-based milk replacer formulation for bovines comprising lactase according to any one of the embodiments 6-11, wherein the dairy-based milk replacer formulation comprising lactase comprises a lower lactose level than the dairy-based milk replacer formulation for bovines in which the lactase is omitted, comprising the steps of:
   (a) providing a dairy-based milk replacer formulation for bovines comprising at least 20 wt.% lactose based on dry matter;
   (b) providing lactase;
   (c) mixing the dairy-based milk replacer formulation of step (a) with the lactase of step (b) and incubating for at least 5 minutes, preferably 5 minutes - 60 minutes, more preferably 10 minutes - 45 minutes, at a temperature of 20°C - 60°C, preferably 25°C - 50°C, more preferably 35°C - 45°C.

### Example 1 - In Vitro Experiments

### Bacterial strains and growth conditions

The *C*. *perfringens* type A strain used in this study (BCP20) was a clonal population, isolated from abomasal and intestinal ulcers of a calf that was diagnosed with bovine necro-haemorrhagic enteritis (Van Immerseel et al. 2010). The strain was grown overnight (ON) in 10 mL TGY broth (3% tryptone, 2% yeast extract, 0.1% glucose, 0.1% L-cysteine) under anaerobic conditions at 40°C.

### In vitro assay for Clostridial growth

Lactose, casein and whey protein isolate (WPI) were added to TGY medium at concentrations corresponding to their relative presence in skimmed milk powder on a dry matter (DM) basis. Ten- and 100-fold dilutions of these concentrations were also applied, to gain a 'high', 'intermediate' and 'low' level for each supplement (Table 1). Furthermore, the saccharide component (lactose, glucose, galactose) was tested similarly, at concentrations as shown in Table 2.

**Table 1. Supplement concentrations of skimmed milk powder and subcomponents at 10-, 100- and 1000-fold dilutions (high, intermediate and low, respectively) in TGY-medium at concentrations corresponding to their relative presence in skimmed milk powder on a dry matter (DM) basis.**

| | | **Skimmed milk powder** | **Lactose** | **Casein^{a}** | **Whey protein ^{b}** |
|---|---|---|---|---|---|
| In skimmed MP | | 100% | 50% | 28% | 7.00% |
| In medium | High | 10.0% | 5.0% | 2.8% | 0.7% |
| | Intermediate | 1.0% | 0.5% | 0.28% | 0.07% |
| | Low | 0.1% | 0.05% | 0.028% | 0.007% |
| | Control | 0.0% | 0.0% | 0.0% | 0.0% |

| | | | | | |
|---|---|---|---|---|---|
| ^{a}Assuming 80% of crude protein (CP) in skimmed milk powder is casein ^{b}Assuming 20% of CP in skimmed milk powder is whey protein | | | | | |

**Table 2. Concentrations of tested saccharides (lactose, glucose, galactose) in TGY medium.**

| | **Supplement concentration ^{a}** | | | |
|---|---|---|---|---|
| | **mM** | | **g/L** | |
| Lactose | 14.61 | | 5.00 | |
| Lactose (lactase treated ^{b}) | 14.61 | | 5.00 | |
| Glucose | 14.61 | | 2.63 | |
| Galactose | 14.61 | | 2.63 | |
| Glucose and galactose | 7.31 | 7.31 | 1.32 | 1.32 |

| | | | | |
|---|---|---|---|---|
| ^{a} Concentrations of saccharides were adjusted to ensure equimolarity. ^{b} Lactase-treatment 220 U/L, incubated overnight at 40°C | | | | |

A *C. perfringens* BCP20 ON culture was diluted (1:10 000) in each of the supplemented TGY broths, followed by incubation for 24 hours (anaerobically, 40°C). During this incubation, Clostridial growth was determined at 0, 1, 2, 3, 4, 5, 6.5, 8 and 24 hours, by standard plate count of colony forming units (CFU) on Columbia agar plates (Oxoid, Basingstoke, UK) supplemented with five per cent defibrinated sheep blood. The plates were incubated overnight (anaerobically, 40°C).

For the measurement of alpha toxin activity, 2 mL aliquots of the supplemented media were taken after five hours of growth, and centrifuged to obtain cell-free supernatants. The supernatants were filtrated through a 0.22 µm filter, and stored at -20°C until further use.

### Alpha toxin activity

An egg yolk diffusion assay was performed to determine alpha toxin activity in the supernatants as previously described (Rigby, 1981). Briefly, Columbia agar (Oxiod, Basingstoke, UK) was supplemented with two per cent (volume/volume) egg yolk and round holes (diameter of 7 mm) were stabbed in the agar plates, using the back of 200 µL pipette tips. Twenty microliters of the tested supernatants were added to the holes. Plates were incubated at 37°C for 24 hours and scanned with a GS-800 calibrated densitometer (Bio Rad Laboratories, Hercules, CA). Alpha toxin breaks down the egg yolk lecithin in the plate, thereby creating a dense area around the spotted supernatants. The magnitude of the area is correlated to the alpha toxin activity.

The zone of opacity around each spot was measured using Quantity One software (Bio Rad Laboratories) and quantified using a dilution series (1.0, 0.5, 0.25, 0.125, 0.0625 and 0.0313 U/mL) of phospholipase C (Sigma-Aldrich, St. Louis, USA) as a standard. The assay was performed in triplicate.

### Results

### Clostridial growth affected by different nutrient sources

A growth curve was made to determine whether growth of a *C*. *perfringens* type A strain was affected by the type and concentration of the supplement and, to guarantee equal colony forming units (CFU) concentration at the moment of alpha toxin activity determination. To correct for potential differences in concentration of the different ON cultures, data are expressed as the ratio of determined CFU concentration to the starting concentration. No differences of significance were observed for any of the levels of skimmed milk powder and its subcomponents (table 1), nor for the saccharides of table 2. The culture always reached its stationary phase at four hours of incubation, regardless of the type or concentration of every tested supplement. In conclusion, none of the tested supplements affects growth of *C*. *perfringens.*

### Alpha toxin activity

To investigate the effect of all aforementioned supplements and the different concentrations on alpha toxin, supernatants of cultures that were incubated for five hours, were tested in an egg yolk assay. All values are expressed as fraction of the control (un-supplemented TGY medium), which is adjusted to 1.00 U/mL. The results as presented in figure 1 show that both the type of supplement and its concentration affected alpha toxin activity. The observations on skimmed milk powder are in confirmation with the reported data of Valgaeren et al., as adding five percent (w/v) of skimmed milk powder to the medium caused a significant drop in alpha toxin activity (0.40 ± 0.19 U/mL, p<0.001), compared to the control. The same was true for high levels of lactose (0.42 ± 0.70 U/mL, p<0.001) and casein (0.71 ± 0.89 U/mL, p<0.05). Even more, this effect was also observed for the intermediate level of lactose (0.59 ± 0.23 U/mL, p<0.001).

Differences in alpha toxin activity between the different concentrations (high versus intermediate and/or low) within skimmed milk powder and lactose, indicate a concentration-depended effect. No differences were observed between the different levels of casein and WPI. The latter strongly suggests that lactose is the responsible subcomponent of skimmed milk powder for the observed effects.

### Example 2 - In Vivo Experiments

### Experimental setup

The animal experiment was approved by the Ethical Committee of Ghent University (EC 2020-001). Eighteen healthy Holstein-Friesian bull calves (age 16.6 ± 3.3 days; body mass 52.7 ± 1.6 kg) were all transported from the same dairy farm to the Faculty of Veterinary Medicine, Ghent University. Immediately after arrival they were randomly assigned into two test groups (A, B) by coloured ear tags. Animals from both groups were equally divided over three stables of 21.4, 21.4 and 20.4 m2. During the first three weeks after arrival, animals were kept individually, thereafter grouped per three. The concrete floors were bedded with rubber mats, which were cleaned on a bidaily basis. Animals were fed three times a day at 09h00, 15h00 and 20h00, with a milk replacer that contained 45% lactose on a dry matter (DM) basis (Table 3, Nukamel, Weert, The Netherlands), following a feeding regime in which the milk replacer volume increased during the first month. The feeding amount started at four litres per day in the first week, which then every week increased with two litres, to reach the maximum amount of ten litres per day, from the fourth week until the end of the experiment. The daily amount was always equally divided over the three feeding moments.

Milk was prepared as instructed by the manufacturer, and either fed untreated (group A) or provided with 220 U lactase per litre milk (Disolut, Donkerbroek, The Netherlands) (group B). The required lactase concentration was determined by testing different concentrations throughout time, using a lactose assay kit (Sigma-Aldrich, St. Louis, USA) to verify the minimal required incubation time and lactase concentration to remove all present lactose, which was 15 minutes and 110 U/L, respectively. After addition of the enzyme, milk was stirred for at least 20 minutes at 40°C to allow complete digestion of the lactose present in the milk using a milk shuttle (Holm & Laue, Westerronfeld, Germany). Until the age of four weeks, animals consumed only the milk replacer. Thereafter they also had ad libitum access to clean drinking water.

Serum samples were taken every two weeks, up until 12 weeks after arrival, and kept at room temperature (RT) to allow clotting. Subsequently, serum was separated and stored frozen (-20°C) until further processing. Always directly after blood sampling, a faecal sample was taken rectally and stored at -20°C.

**Table 3. Nutritional composition of fed milk replacer**

| **Nutrient analysed (wt.%)^{a}** | |
|---|---|
| Dry matter (DM) | 15 |
| Crude protein | 23.5 |
| Crude fiber | 0.01 |
| Ether extract | 18 |
| Crude ash | 7.0 |
| Lactose | 45 |

| | |
|---|---|
| ^{a} *DM (% as fed), all other nutrients (% dry matter)* | |

### Alpha toxin antibody production

Serodiagnosis of *C*. *perfringens* alpha toxin was performed using a blocking ELISA test (K291, Bio-X, Belgium). The test was performed as per the manufacturer's instructions.

### Relative presence of C. perfringens

To quantify the relative presence of *C*. *perfringens* in the samples throughout the study, DNA was extracted from 100 mg faeces by performing the CTAB method as described by Griffiths et al. (2000), with modifications (Aguirre et al. 2019). DNA was then eluted in 100 µL water and the concentration was thereafter measured with a Nanodrop 1000 spectrophotometer and diluted to 50 ng/µL. Total bacteria and C. perfringens were quantified by performing qPCR, targeting the 16S rRNA gene and cpa gene, respectively. To determine the number of total bacteria, primers Uni_F (5'-ACTCCTACGGGAGGCAGCAG-3') [SEQ ID NO: 1] and Uni_Rev (5'-TTACCGCGGCTGCTGG-3') [SEQ ID NO: 2] were used (Mountzouris et al. 2015). The number of gene copies encoding the cpa gene was quantified using primers AlphaFW (5'-GTTGATAGCGCAGGACATGTTAAG-3') [SEQ ID NO: 3] and AlphaRev (5'-CATGTAGTCATCTGTTCCAGCATC-3') [SEQ ID NO: 4]. qPCR was performed using SYBR-green 2x master mix (Bioline, Brussels, Belgium) in a Bio-Rad CFX-96 system. For the reaction a 12 µL total reaction mixture was used, consisting of 2 µL of the DNA sample and 0.5 µM final qPCR primer concentration. Each reaction was performed in triplicate. The qPCR conditions used were similar for both genes, except for the final step: one cycle at 95°C for ten minutes, 40 cycles of 95°for 30 seconds, then 60°C for 1 minute, and finally (for the 16s rRNA gene) a stepwise temperature increase (60°C to 95°C) at 5s per 0.5°C. For the cpa gene, the final stepwise temperature increase was 65°C to 95°C at 10s per 0.5°C. For construction of the standard curve, the PCR product was generated using the following standard PCR primers '5-ctggcggcgtgcttaacacatg-3' (forward) [SEQ ID NO: 5] and '5-gcccagtaaatccggataac-3' (reverse) [SEQ ID NO: 6] for total bacteria, and '5-agtctacgcttgggattgaa-3' (forward) [SEQ ID NO: 7] and '5-tttcctgggttgttcaattc-3' [SEQ ID NO: 8] for *C. perfringens.* The concentration of the linear dsDNA standard was adjusted to ten-fold dilutions from 1×107 to 1×101 copies per µL. The copy numbers of samples were determined by reading off the standard series with the Ct values of the samples. Finally, relative presence of C. perfringens was calculated by dividing the absolute amount of C. perfringens by the total amount of bacteria.

### Statistical analysis

To compare alpha toxin inhibition in both groups, unpaired T-tests were performed for every time point, with test group as fixed factor. Possible interactions between group and sampling moment were included in the model. Figures were made using GraphPad Prism 5.

### Results

### Relative presence of C. perfringens in faecal samples

The faecal samples that were collected throughout the *in vivo* trial of the present research were used to determine absolute counts of total bacteria and *C. perfringens*, to quantify the relative presence of *C*. *perfringens.* Throughout the whole study, there were no large differences between the two test groups (Figure 2). The samples from the last time point showed a significant difference (p = 0.011) between both groups, but was not biologically relevant (7.70E-08%). Also the absolute amount of bacteria and of *C*. *perfringens* was not significantly different between the calves that received no lactase and the calves that received lactase (data not shown). Strikingly both test groups demonstrated an equally low occurrence of diarrhoea, even though the osmolality of the lactose-split diet is higher.

### Antibody production against C. perfringens alpha toxin

The antibody production against alpha toxin was determined for each time point by ELISA (Table 3). After six weeks, there started to be a clear distinction between both groups. While antibody levels in the serum of calves from the 'lactase provided' group remained high, they started to decline in calves that did not receive lactase. The decline created an increasing discrepancy between the two groups, which was 9.4% inhibition (p<0.001) at 8 weeks and 15.0% inhibition (p<0.001) at 12 weeks (Figure 3). The difference is indicative for a build-up of active immunity against *C*. *perfringens* related conditions for the 'lactase provided' group, whilst this build-up is poor in the calves that did not receive lactase.

### Example 3 - Larger scale in vivo experiment

EXAMPLE OUTLINE IN BRIEF: A test at a commercial veal production facility under conditions common for the industry is performed. Eighty calves are selected with an age of about 19.3 ± 5.5 days with normal maternal (so passive) antibody titre. The animals are housed in regular housing and their location in the housing is regularly interchanged in order to prevent any influence of the housing. The group is split in two with one group receiving regular milk replacer with high lactose level whilst the other group receives the same milk replacer provided with lactase. A common feeding schedule for white veal calves is followed and the feed intake is monitored. On regular basis blood and faeces samples are taken and analysed for antibodies, *C*. *perfringens* and the microbiome (16S next generation sequencing).

### EXAMPLE OUTLINE IN DETAIL:

### Materials and Methods

### Diet: a high lactose milk replacer versus a lactose-free equivalent

To determine the effect of lactose on the alpha toxin antibody development, calves were fed either a high lactose milk replacer ('Lact'; n = 40) or a lactose-free milk replacer ('NoLact'; n = 40). Throughout the trial, three different milk replacers were used; on average they contained 44.4 % lactose (on dry matter (DM) basis). All were produced by Nukamel (Table 4; Nukamel, Weert, The Netherlands) and prepared as instructed by this manufacturer. Hundred forty five (145) gram of the milk replacer was dissolved per liter of water. In order to obtain a lactose-free milk replacer, without affecting any other components, the Lact milk replacer was treated with lactase (Sternenzym, Ahrensburg, Germany). To identify the minimal required incubation time and lactase concentration needed to digest all lactose in the high lactose milk replacer, the milk was treated with different lactase concentrations (ranging from 0 to 2000 U/L) at different incubation times (ranging from 0 to 90 minutes) at 42°C. The residual lactose concentration present in the milk after lactase treatment was determined using a lactose assay kit (Sigma-Aldrich). A lactase concentration of 1000 U/L was used to degrade all lactose within 30 minutes of incubation at 42°C. To avoid the presence of any residual lactose in the lactose-free milk, in the subsequent animal treatment trial, the milk replacer was treated with a two-fold concentration of lactase (2000 U/L) for at least 40 minutes at 42°C.

### Feeding regime

Animals were fed two times a day at 06h00 and 18h00, following an increasing feeding regime in which the milk replacer volume as defined by Nukamel. First, the total amount of milk was prepared for all animals in a large milk tank. Thereafter, half of the milk was transferred to a milk shuttle to ensure equal conditions for mixing time and temperature, throughout the whole trial. Lactase was added to this remainder amount of milk. The milk was then mixed for at least 40 minutes at 42°C to allow complete hydrolyzation of lactose, and offered at the same temperature.

Concentrate (Table 8, Bepro NV, Belgium) was fed according to a standardized feeding schedule. At the start the calves received 50 gram concentrate per animal per day. This amount increased weekly with 50 gram for the subsequent eight weeks. Thereafter, the amount was kept constant at 500 gram per animal per day. During the last month of the trial, slightly less concentrate was fed (450 gram in week 25 and 400 gram in the weeks thereafter).

### Animal selection and housing conditions

The animal experiment was approved by the Ethical Committee of Ghent University (EC 2021-027). Hundred Holstein-Friesian male bull calves (body weight 51.1 ± 4 kg) were selected from veal distribution centre (Vilatca, Belgium) 19.3 ± 5.5 days old. From there, they were transported to a farm in Merksplas, Belgium. One day after arrival, the calves were weighed and blood and faecal samples were taken. The next day, a blocking ELISA test (K291, Bio-X Diagnostics, Rochefort, Belgium) was performed to determine antibody concentration against alpha toxin, expressed in percentage inhibition of alpha toxin. The test was performed as per the manufacturer's instructions. With respect to the maternal antibody titers against alpha toxin, the twenty most divergent calves were removed from the group to gain a uniform test population. The eighty remaining calves were randomly divided into two test groups by the appliance of coloured ear tags: calves that consumed regular, untreated milk replacer (Lact; n = 40) and those that consumed lactase-treated milk replacer (NoLact; n = 40).

Calves were housed in a well ventilated stable with a wooden slatted floor. During the first six weeks after arrival, they were kept individually ( 1.8 m²), thereafter grouped per four ( 8.9 m²). At group housing, they had *ad libitum* access to clean drinking water.

### Collection of serum and faeces

After the animals were assigned to their respective test group, blood and faeces were sampled every two weeks. Body weight was measured up until the eighth week. The blood samples were taken using vacutainer serum tubes and then left at room temperature to allow clotting. After transportation to the Faculty of Veterinary Medicine of Ghent University (Belgium), serum was transferred into 2 mL eppendorf tubes and stored at -20°C until further use. The faecal samples were obtained from the rectum, transported on ice and kept at 4°C overnight.

### Determination of Clostridial growth

Faecal samples were used to detect and quantify *Clostridium perfringens.* For this, faecal samples were inoculated on selective agar plates (CHROMagar TM, Paris, France), using inoculation loops. The plates were then incubated overnight (40°C, anaerobic environment). The next day, the plates were visually inspected: samples that suggested any sign of Clostridial growth, were subjected to quantification of *C*. *perfringens.* This was done by standard plate count of colony forming units (CFU) on aforementioned selective CHROMagar plates. For this, faeces was mixed in PBS (1:10, w/v) and thoroughly vortexed. Thereafter, it was diluted 10-, 100- and 1000 times in PBS, and both diluted and undiluted samples were spotted on the plates (six spots, 20 µL). After drying, the plates were incubated overnight (anaerobically, 40°C), followed by a colony count and calculated to CFU per gram faeces.

### Determination of antibody titers against Clostridium perfringens alpha toxin

Sero-diagnosis of *C*. *perfringens* alpha toxin was performed on the serum samples, using the K291 blocking ELISA commercial test kit (K291, Bio-X, Belgium), as outlined in Example 2. The test was performed as per the manufacturer's instructions.

**Table 4 - Milk replacer composition**

| **Composition** | **START** | **Mid** | **END** |
|---|---|---|---|
| Skimmed milk powder (%) | 30 | 15 | 30 |
| Whey powder (%) | 44.4 | 52.8 | 43.9 |
| Wheat concentrate pregelatinized (%) | 0 | 0 | 0 |
| hydrolyzed wheat protein concentrate | | | |
| (%) | 6.5 | 7.5 | 2.1 |
| soy protein concentrate (SPC) (%) | 0 | 2 | 2.5 |
| Coconut oil (%) | 5 | 5.7 | 4.3 |
| Palm oil (%) | 1 | 4 | 6.3 |
| Lard (%) | 6 | 6 | 5 |
| Tallow (%) | 3.7 | 2.1 | 2 |
| Soy lecithin (%) | 1 | 1 | 1 |
| Emulsifier (%) | 0.8 | 0.8 | 0.8 |
| Premix (vitamins, trace elements, | | | |
| minerals, amino acids) (%) | 1.5 | 3.1 | 2.3 |
| | | | |

| **Analytical constituents** | | | |
|---|---|---|---|
| protein (%) | 22.5 | 21 | 20 |
| fat (%) | 18.5 | 20 | 20 |
| lactose (%) | 44.5 | 43.9 | 44.8 |
| ash (%) | 6.9 | 7 | 7.1 |
| fiber (%) | 0.02 | 0.1 | 0.1 |
| lysine (%) | 1.9 | 1.8 | 1.7 |
| methionine (%) | 0.5 | 0.5 | 0.4 |
| threonine (%) | 1.1 | 1 | 1 |
| calcium (%) | 0.8 | 0.8 | 0.8 |
| phosphorus (%) | 0.7 | 0.7 | 0.7 |
| sodium (%) | 0.5 | 0.5 | 0.5 |
| chlorine (%) | 1.1 | 1.2 | 1.1 |
| potassium (%) | 1.6 | 1.5 | 1.6 |
| ME calf (MJ) | 18.6 | 18.8 | 18.4 |
| Trace elements and vitamins | | | |
| Magnesium total (%) | 0.15 | 0.14 | 0.15 |
| Magnesium added (%) | 0.03 | 0.03 | 0.03 |
| Fe total (mg/kg) | 98.63 | 12.99 | 12.25 |
| Fe sulphate monohydrate (3b103) (mg/kg) | 90.75 | | |
| Cu total (mg/kg) | 9.19 | 9.42 | 9.43 |
| Cu sulphate pentahydrate (3b405) (mg/kg) | 8.1 | 8.1 | 8.1 |
| Zn total (mg/kg) | 59.01 | 161.6 | 169.94 |
| Zn sulphate monohydrate (3b605) (mg/kg) | 40 | 150 | 150 |
| Mn total (mg/kg) | 22.85 | 38.54 | 38.64 |
| Mn sulphate monohydrate (3b503) (mg/kg) | 20 | 35 | 35 |
| I total (mg/kg) | 1.80 | 1.78 | 1.80 |
| I K-iodide (3b201) (mg/kg) | 1.36 | 1.36 | 1.36 |
| Se total (mg/kg) | 0.46 | 0.47 | 0.45 |
| Sodium selenite (3b801) (mg/kg) | 0.4 | 0.4 | 0.4 |
| Vit A add (3a672a) (IE/kg) | 25,000 | 25,000 | 25,000 |
| Vit D3 add (3a671) (IE/kg) | 4,000 | 4,000 | 4,000 |
| Vit E add (3a700) (mg/kg) | 150 | 150 | 150 |
| Vit B1 add (3a821) (mg/kg) | 10 | 10 | 10 |
| Vit B2 add (mg/kg) | 6 | 6 | 6 |
| Vit B6 add (3a831) (mg/kg) | 7 | 7 | 7 |
| Vit B12 add (µg/kg) | 80 | 80 | 80 |
| Vit C Ascorbic acid add (3a300) (mg/kg) | 150 | 150 | 150 |
| Vit K (mg/kg) | 2 | 2 | 2 |
| Pantothenic acid add (mg/kg) | 30 | 30 | 30 |
| Folic acid add (3a316) (mg/kg) | 1 | 1 | 1 |

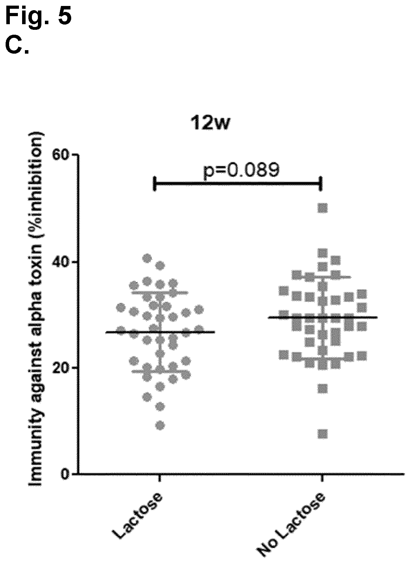

Between 10 and 12 weeks after start of the trial, there was a significant reduction (5.0%, p = 0.002; Figure 4, Figure 5A, Figure 5C, Table 5) of antibody concentration in the 'Lactose' group, whereas values of the 'No Lactose' group remained constant (p = 0.47; Figure 4, Figure 5B, Figure 5C, Table 5). The resulting difference in alpha-toxin inhibition between both groups was 2.71% (Table 6, Table 7). This provides insight into the pathogenesis of necro-haemorrhagic enteritis, as well as insight into the susceptibility of calves towards necro-haemorrhagic enteritis, and as a consequence provides insight into the current mortality rates in the veal sector. Moreover, these results of the trial show that feeding the calves with milk replacer comprising a lower content of lactose than regular milk replacer currently fed to calves, provides the benefit of preventing the decrease in anti-alpha toxin antibody titer expressed as higher immunity against alpha toxin (% inhibition; Figure 4, Figure 5, Tables 5-7). Depletion of lactose in the feed for the calves thus aids in maintaining immunity against alpha-toxin at a higher level than for calves that are fed milk replacer containing a higher amount of lactose. (Pre-)Treatment of milk replacer with lactase is a suitable method for provision of milk replacer with a sufficiently reduced lactose content, wherein said milk replacer after lactase treatment is endowed with the capacity to influence the calfs' anti-alpha toxin antibody titer such that the titer is higher than the titer for calves fed with milk replacer non treated with lactase and therefore comprising a higher content of lactose.

**Table 5 - Antibody titers between 10 and 12 weeks after start of the trial, showing a decline in anti-alpha toxin antibody concentration for the lactose group. The p-value concerns the statistical relevance of difference between week 10 and week 12.**

| | **Lactose** | | **No lactose** | |
|---|---|---|---|---|
| | *Mean* | *SD* | *Mean* | *SD* |
| Week 10 (W10) | 32.2 | 6.9 | 31.0 | 6.7 |
| Week 12 (W12) | 27.2 | 6.9 | 29.9 | 7.0 |
| Difference | 5.0 | | 1.1 | |
| p-value | **0.002** | | 0.471 | |

**Table 6 - Summarizing table of the antibody titers from 8 to 12 weeks after the start of the trial for calves that consumed milk replacer (n = 40) and calves that received a lactose-free equivalent (n = 40)**

| **weeks** | **Lactose** | **No lactose** | **difference** | **p-value** |
|---|---|---|---|---|
| 8w | 32.6 | 32.0 | 0.53 | 0.68 |
| 10w | 32.2 | 31.0 | 1.16 | 0.45 |
| 12w | 27.2 | 29.9 | 2.71 | 0.089 |

**Table 7 - Summarizing table of the antibody titers from 8 to 12 weeks after the start of the trial for calves that consumed milk replacer (n = 40) and calves that received a lactose-free equivalent (n = 40)**

| | **8 weeks** | | **10 weeks** | | **12 weeks** | |
|---|---|---|---|---|---|---|
| | *Mean* | *SD* | *Mean* | *SD* | *Mean* | *SD* |
| Lactose | 32.6 | 7.5 | 32.2 | 6.9 | 27.2 | 6.9 |
| No Lactose | 32.0 | 8.1 | 31.0 | 6.7 | 29.9 | 7.0 |
| Difference | 0.53 | | 1.16 | | 2.71 | |
| p-value | 0.68 | | 0.45 | | 0.089 | |

**Table 8 - Concentrate composition**

| **Composition** | |
|---|---|
| Barley | |
| Lupins | |
| Maize | |
| Corn flakes | |
| Molasses | |
| Oats | |
| Wheat | |
| Corn gluten | |
| Hydrogenated palm oil | |
| Crude soybean oil | |

| **Analytical constituents** | |
|---|---|
| protein (%) | 15.5 |
| fat (%) | 3.8 |
| ash (%) | 3.5 |
| fiber (%) | 6.5 |
| calcium (%) | 0.24 |
| phosphorus (%) | 0.31 |
| sodium (%) | 0.1 |

| **Trace elements and vitamins** | |
|---|---|
| Zn sulphate monohydrate (3b605) (mg/kg) | 96 |
| Cu (II) sulphate (3b405) (mg/kg) | 6 |
| Mn sulphate monohydrate (3b503) (mg/kg) | 40 |
| I K-iodide (3b201) (mg/kg) | 0.7 |
| Sodium selenite (3b801) (mg/kg) | 0.2 |
| Vit A add (3a672a) (IE/kg) | 9700 |
| Vit D3 add (3a671) (IE/kg) | 1933 |

| **Probiotics** | |
|---|---|
| Saccharomyces cerevisea NCYC Sc | |
| 47 (4b1702) (CFU/kg) | 5×10⁹ |

## Claims

1. Lactase for use in any one or more of: preventing an alimentary tract disorder caused by *Clostridium perfringens* in a bovine subject, preventing a *Clostridium perfringens* alpha-toxin related alimentary tract disorder in a bovine subject, increasing active immunity against an alimentary tract disorder in a bovine subject caused by *Clostridium perfringens,* increasing active immunity against a *Clostridium perfringens* alpha-toxin related alimentary tract disorder in a bovine subject and stimulating *Clostridium perfringens* alpha-toxin antibody production in a bovine subject, wherein the lactase is orally administered to the bovine subject.

2. Lactase for use according to claim 1, wherein the *Clostridium perfringens* alpha-toxin related bovine alimentary tract disorder or the bovine alimentary tract disorder caused by *Clostridium perfringens* is any one or more selected from the list consisting of enterotoxaemia, necro-haemorrhagic enteritis, clostridial abomasitis, haemorrhagic enteritis, haemorrhagic abomasitis and abomasal ulceration, preferably the *Clostridium perfringens* alpha-toxin related bovine alimentary tract disorder or the bovine alimentary tract disorder caused by *Clostridium perfringens* is necro-haemorrhagic enteritis and/or enterotoxaemia, more preferably necro-haemorrhagic enteritis.

3. Lactase for use according to claim 1 or 2, wherein the lactase is provided as a single dosage form for oral application, preferably as a tablet, powder, capsule or solution for oral use.

4. Lactase for use according to any one of the claims 1-3, wherein the lactase is administered to a bovine subject who is fed a lactose-comprising diet, and wherein optionally the lactase is administered to the bovine subject before, during or after the bovine subject is fed the lactose-comprising diet, preferably 5 seconds - 5 minutes before, during or 5 seconds - 5 minutes after the bovine subject is fed with the lactose-comprising diet, more preferably during the bovine subject is fed with the lactose-comprising diet, and/or wherein optionally the lactose-comprising diet is selected from any one of: a milk, preferably bovine milk and a milk replacer for bovines, preferably a dairy-based milk replacer for bovines.

5. Lactase for use according to any one of the claims 1-3, wherein the lactase is provided in a dairy-based milk replacer formulation for bovines, preferably for cattle, more preferably for calves, wherein the dairy-based milk replacer formulation comprises at least 10 wt.% lactose based on dry matter, preferably at least 30 wt.% lactose based on dry matter, more preferably at least 40 wt.% lactose based on dry matter, and wherein optionally the dairy-based milk replacer formulation for bovines is a liquid, a powder, a granulate or a pellet, preferably a liquid.

6. Lactase for use according to claim 5, wherein the dairy-based milk replacer formulation is fed to the bovine subject after a waiting time of 5 minutes or more after providing the lactase to the dairy-based milk replacer formulation, preferably 5 - 15 minutes wherein the dairy-based milk replacer formulation during the waiting time has a temperature of 20 - 60°C; and wherein in case the dairy-based milk replacer formulation is a powder, a granulate or a pellet, the powder, granulate or pellet has been dissolved before the waiting time.

7. Lactase for use according to claim 5 or 6, wherein the dairy-based milk replacer formulation comprises 10 - 35 wt.% protein based on total dry weight of the milk replacer, 10 - 30 wt.% lipids based on total dry weight of the milk replacer formulation and 30 - 60 wt.% of digestible carbohydrates based on total dry weight of the milk replacer formulation.

8. Lactase for use according to any one of the claims 5-7, wherein the dairy-based milk replacer formulation comprises 10 - 60 wt.% lactose based on dry matter, preferably 20 - 55 wt.%, more preferably 30 - 50 wt.%.

9. Lactase for use according to any one of the claims 5-8, wherein lactase is provided in an amount of 4000 U or more lactase per kg dry weight of the dairy-based milk replacer formulation, preferably 8000 U or more, most preferably 12000 U or more.

10. Lactase for use according to any one of the previous claims, wherein the lactase is administered to the bovine subject starting from 28 days after birth of said bovine subject, preferably starting from 14 day after birth, even more preferably starting from 7 day after birth most preferably starting directly after birth, and/or wherein the lactase is consecutively administered for 20 days or more, preferably for 40 days or more, more preferably for 60 days or more.

11. Lactase for use according to any one of the preceding claims, wherein the bovine subject is a cattle subject, preferably wherein the bovine subject is a calf, even more preferably wherein the bovine subject is a veal calf, a beef calf or a dairy calf, most preferably wherein the bovine subject is a veal calf.

12. Lactase for use according to any one of the preceding claims, wherein lactase is provided in an amount of 2000 - 35000 U lactase per day, preferably an amount of 4000 - 30000 U lactase per day.

13. Lactase for use according to any one of the preceding claims, wherein the active immunity against a bovine alimentary tract disorder caused by *Clostridium perfringens* is increased compared to a bovine subject that is not administered the lactase, the active immunity against a *Clostridium perfringens* alpha-toxin related bovine alimentary tract disorder is increased compared to a bovine subject that is not administered the lactase and/or the *Clostridium perfringens* alpha-toxin antibody production in bovines is stimulated compared to a bovine subject that is not administered the lactase.

14. Use of lactase for any one or more of: preventing a bovine alimentary tract disorder caused by *Clostridium perfringens,* preventing a *Clostridium perfringens* alpha-toxin related alimentary tract disorder in the bovine subject, increasing active immunity against an alimentary tract disorder in the bovine subject caused by *Clostridium perfringens,* increasing active immunity against a *Clostridium perfringens* alpha-toxin related alimentary tract disorder in the bovine subject, and stimulating *Clostridium perfringens* alpha-toxin antibody production in the bovine subject, wherein the lactase is orally administered to the bovine subject.

15. Use of lactase according to claim 14, wherein the lactase is administered to a bovine subject who is fed a lactose-comprising diet, wherein optionally the lactose-comprising diet is selected from any one of: a milk, preferably bovine milk, a milk replacer for bovines, preferably a dairy-based milk replacer for bovines.

16. Use of lactase according to claim 14 or 15, wherein the lactase is provided as a dairy-based milk replacer formulation for bovines comprising at least 10 wt.% lactose based on dry matter, preferably at least 30 wt.% lactose based on dry matter, more preferably at least 40 wt.% lactose based on dry matter.

17. Use of lactase according to any one of the claims 14-16, wherein the bovine subject is a cattle subject, preferably wherein the bovine subject is a calf, even more preferably wherein the bovine subject is a veal calf, a beef calf or a dairy calf, most preferably wherein the bovine subject is a veal calf.

18. Method for providing a dairy-based milk replacer formulation for bovines comprising lactase according to any one of the claims 5-9, wherein the dairy-based milk replacer formulation comprising lactase comprises a lower lactose level than the dairy-based milk replacer formulation for bovines in which the lactase is omitted, comprising the steps of:
(a) providing a dairy-based milk replacer formulation for bovines comprising at least 20 wt.% lactose based on dry matter;
(b) providing lactase;
(c) mixing the dairy-based milk replacer formulation of step (a) with the lactase of step (b) and incubating for at least 5 minutes, preferably 5 minutes - 60 minutes, more preferably 10 minutes - 45 minutes, at a temperature of 20°C - 60°C, preferably 25°C - 50°C, more preferably 35°C - 45°C.


SEQUENCE LISTING

<110>  Nukamel NV

<120>  LACTASE FOR USE

<130>  P6100026EP

<150> NL 2026691
<151> 2020-10-16

<150> NL 2028319
<151> 2021-05-28

<160>  8

<170>  PatentIn version 3.5

<210>  1
<211>  20
<212>  DNA
<213>  Artificial Sequence

<220>
<223>  Synthetic oligonucleotide primer 1

<400>  1
actcctacgg gaggcagcag                                                    20


<210>  2
<211>  16
<212>  DNA
<213>  Artificial Sequence

<220>
<223>  Synthetic oligonucleotide primer 2

<400>  2
ttaccgcggc tgctgg                                                        16


<210>  3
<211>  24
<212>  DNA
<213>  Artificial Sequence

<220>
<223>  Synthetic oligonucleotide primer 3

<400>  3
gttgatagcg caggacatgt taag                                               24


<210>  4
<211>  24
<212>  DNA
<213>  Artificial Sequence

<220>
<223>  Synthetic oligonucleotide primer 4

<400>  4

catgtagtca tctgttccag catc                                              24

<210>  5
<211>  22
<212>  DNA
<213>  Artificial Sequence

<220>
<223>  Synthetic oligonucleotide primer 5

<400>  5
ctggcggcgt gcttaacaca tg                                                22

<210>  6
<211>  20
<212>  DNA
<213>  Artificial Sequence

<220>
<223>  Synthetic oligonucleotide primer 6

<400>  6
gcccagtaaa tccggataac                                                   20

<210>  7
<211>  20
<212>  DNA
<213>  Artificial Sequence

<220>
<223>  Synthetic oligonucleotide primer 7

<400>  7
agtctacgct tgggattgaa                                                   20

<210>  8
<211>  20
<212>  DNA
<213>  Artificial Sequence

<220>
<223>  Synthetic oligonucleotide primer 8

<400>  8
tttcctgggt tgttcaattc                                                   20